# EUROPEAN PATENT APPLICATION

(11) **EP 4 748 817 A1**
(43) Date of publication of application: **27.05.2026**
(21) Application number: 24214985.4
(22) Date of filing: 23.11.2024
(51) Int. Cl.: C07C 1/04, C07C 1/12, C07C 9/04, C07C 11/02, C07C 29/151, C07C 31/04, B01D 53/62

(54) **SYSTEM COMPRISING CO2 ADSORPTION UNIT COUPLED WITH THERMAL RECOVERY OXYFUEL COMBUSTOR AND PROCESS FOR UTILIZATION OF THE CO2**

(71) Applicant: ICODOS GmbH, 68163 Mannheim (DE)
(72) Inventor: Schütz, Michael Josef, 68163 Mannheim (DE); Vidal Vázquez, Francisco de Sales, 68163 Mannheim (DE); de la Flor Sánchez, Daniel, 68163 Mannheim (DE)
(74) Representative: ZBM Patents - Zea, Barlocci & Markvardsen

(57) **Abstract**

The present invention provides a system and a process for the production of methane, C2-C100 linear alkanes, C2-C10 olefins, oxygenates, methanol and combinations thereof, comprising: an oxyfuel combustor comprising a combustion gas outlet, and a first fluid entrance; a heat recovery system; a carbon capturing unit comprising an adsorption column which comprises a combustion gas inlet in fluid communication with the combustion gas outlet of the oxyfuel combustor, and a feed gas inlet; a desorption column; one or more methanol synthesis reactors; wherein the adsorption column comprises a base portion and a top portion; the adsorption column extends a height from the base portion to the top portion; the base portion corresponds to one half of the height of the adsorption column; the feed gas inlet and the combustion gas inlet are located in the base portion; and the feed gas inlet is located above the combustion gas inlet in the base portion.

## Description

### Technical Field

The present invention belongs to the field of carbon dioxide utilization. In particular, the invention relates to a system for the production of a product selected from the group consisting of methane, C2-C100 linear alkanes, C2-C10 olefins, oxygenates, methanol and combinations thereof, comprising an oxyfuel combustor and a carbon capturing unit, and to a process for the production of a product selected from the group consisting of methane, C2-C100 linear alkanes, C2-C10 olefins, oxygenates, methanol and combinations thereof with a system comprising an oxyfuel combustor and a carbon capturing unit.

### Background Art

In recent years, carbon capturing systems have been developed as means to address the environmental impact of industrial processes. Carbon capture relates to the process of capturing CO₂ from waste emissions, which can then be either stored or utilized in chemical processes. In the production of chemicals, carbon capture can play a vital role by intercepting CO₂ from the exhaust gases or feed, which can then be recycled back into a synthesis process of chemicals such as methanol or methane. This integration of carbon capture into synthetic processes not only reduces greenhouse gas emissions but also enhances the overall efficiency and sustainability of the processes by reusing CO₂ as a feedstock.

However, several challenges persist in the current state of chemical production and carbon capture integration. Waste gases from chemical synthesis often contains unreacted CO₂, carbon monoxide (CO), hydrogen, and other gases that require effective separation technologies to recycle efficiently. Furthermore, there is a growing need for systems that not only reduce greenhouse gas emissions but also operate with high energy efficiency. Current processes demand substantial energy input, primarily due to the high pressures and temperatures needed for the catalytic reactions, which increases operational costs and the carbon footprint of the production process.

As an example, methanol is a widely used chemical with applications in fuels, plastics, and as a feedstock for many industrial chemicals. Traditional methanol production processes primarily involve the catalytic conversion of natural gas or syngas into methanol. This process typically operates at high temperatures and pressures and often relies on a catalytic reaction involving copper-based catalysts. Despite its established role in industrial applications, the production of methanol is energy-intensive and leads to the emission of significant amounts of greenhouse gases, such as carbon dioxide (CO₂).

WO2020048809A1 describes a method for producing methanol from a carbonaceous feedstock, in which method synthesis gas is generated therefrom in a synthesis gas generation unit, the synthesis gas is converted into methanol in a methanol synthesis unit and the obtained reaction mixture is reprocessed in different stages in order to isolate the methanol. Using an oxygen-containing gas, the components carbon monoxide, carbon dioxide, dimethyl ether and methane are burnt off the streams separated during the isolation of the methanol. Then, the carbon dioxide of the resulting flue gas is separated within a carbon dioxide recovery unit and is returned to the syngas generation unit and/or to the methanol synthesis unit.

However, the challenge remains to improve the selectivity toward carbon dioxide capture in carbon capture systems integrated with the production of chemicals. Selectivity is crucial as it determines the system's ability to preferentially capture CO₂ over other gases and reduces carbon footprint. High selectivity not only improves the efficiency of the carbon capture process but also enhances the quality of CO₂ that can be recycled for synthesis. High selectivity can currently only be achieved using advanced materials such as amine-based solvents, membrane technologies or absorbent materials, which can increase the complexity and cost of the system.

Therefore, while existing chemical production technologies and carbon capture systems provide approaches to producing these chemicals sustainably, significant issues remain. These include the need to minimize waste gases, reduce greenhouse gas emissions and improve energy efficiency while enhancing CO₂ selectivity. Addressing these challenges would represent a major advancement in sustainable chemicals production and contribute to broader environmental goals.

### Summary of Invention

The inventors have developed a system, and process for the production of a product selected from the group consisting of methane, C2-C100 linear alkanes, C2-C10 olefins, oxygenates, methanol and combinations thereof, that reduces waste gas emissions and greenhouse gases while achieving efficient energy and material use. The system and process developed by the inventors enable the thermal utilization of waste gases through combustion, along with enhanced selectivity in the adsorption of carbon dioxide (CO₂). Combustion of waste gases in the system according to the invention avoids emissions, increases thermal efficiency and reduces fuel consumption. The system prevents flammable and/or toxic substances from being permanently flared into the atmosphere and valuable CO₂-rich gas from being lost, as CO₂ is utilized for chemicals production.

The inventors surprisingly found that a specific configuration of the inputs and outputs in a system according to the invention comprising an oxyfuel combustor and a carbon capturing unit has a great influence on the selectivity towards CO₂, as well as for complying with the energy requirements of the system.

Improving the selectivity toward carbon dioxide capture has been shown by the inventors to be directly related with the configuration of the fluid's inlets in a carbon capturing unit, which additionally permit the reduction of undesired or inert gases, leading to lower energy consumption. High selectivity in CO₂ capture means that the capture system effectively isolates CO₂ from other gases, such as methane (CH₄); poisoning agents, such as hydrogen sulfide; or inert gases, such as nitrogen (N₂). The gases which do not participate as reactants in the synthesis reaction and often represent a processing burden as they accumulate in the system, increasing the volume of gas that must be managed, separated, or compressed. Reducing the concentration of such gases by increasing the selectivity in CO₂ capture provides several energy-saving benefits.

Firstly, when these gases are minimized, the need for extensive gas recycling loops and energy-intensive separations is reduced. A system with fewer non-reactant or inert gases requires less purging and operates more efficiently, needing less energy to maintain high-pressure and high-temperature conditions necessary for the synthesis. Additionally, lower concentrations of methane or nitrogen reduce the load on compressors, separators, and heat exchangers, directly lowering both capital and operational energy costs.

Moreover, selective CO₂ capture improves the overall reaction efficiency by increasing the partial pressure of the reactant gases (CO₂ and hydrogen), thereby enhancing their conversion rates to the products which can obtained with the system and process according to the invention. In a system filled with non-reactant or inert gases, the partial pressures of CO₂ and hydrogen are reduced, which diminishes the driving force for the synthesis reaction. This leads to decreased reaction rates and necessitates higher energy input to maintain the desired production rate. Furthermore, by selectively capturing CO₂ and minimizing methane and inert gases cycling within the system, the system can operate with higher reactant concentrations, increasing the efficiency of chemicals production, such as methanol, and reducing the overall energy required to achieve the same production output.

Accordingly, a first aspect of the invention relates to a system for the production of a product selected from the group consisting of methane, C2-C100 linear alkanes, C2-C10 olefins, oxygenates, methanol and combinations thereof comprising:
- an oxyfuel combustor comprising a combustion gas outlet, and a first fluid entrance configured to input a carbon dioxide-containing feed gas, a waste gas and an oxygen-containing gas;
- a heat recovery system configured to recover heat from the oxyfuel combustor;
- a carbon capturing unit comprising
   - an adsorption column which comprises a combustion gas inlet in fluid communication with the combustion gas outlet of the oxyfuel combustor, a feed gas inlet configured to input carbon dioxide-containing feed gas, and an overhead outlet configured to output methane and/or nitrogen; and
   - a desorption column which comprises a carbon dioxide outlet configured to either output carbon dioxide-containing synthesis gas or a mixture of carbon dioxide-containing synthesis gas and hydrogen-containing gas;
wherein the adsorption and desorption columns are in fluid communication with each other, the adsorption column is configured to adsorb carbon dioxide, and the desorption column is configured to desorb carbon dioxide;
- one or more synthesis reactors, wherein the one or more synthesis reactors comprise:
   - a synthetized product outlet;
   - a waste gas outlet in communication with the first fluid entrance; and
   - a second fluid entrance configured to input a carbon dioxide-containing synthesis gas and a hydrogen-containing gas, and being in fluid communication with the carbon dioxide outlet;
wherein the adsorption column comprises a base portion and a top portion; the adsorption column extends a height from the base portion to the top portion; the base portion corresponds to one half of the height of the adsorption column; the feed gas inlet and the combustion gas inlet are located in the base portion; and the feed gas inlet is located above the combustion gas inlet in the base portion.

A second aspect of the invention relates to process for the production of a product selected from the group consisting of methane, C2-C100 linear alkanes, C2-C10 olefins, oxygenates, methanol and combinations thereof; from a carbon-dioxide synthesis gas and a hydrogen-containing gas, comprising:
a) conducting in one or more synthesis reactors, a reaction selected from the group consisting of:
   i) methanation, which comprises reacting H2 and CO2 to yield methane, at a temperature ranging from 250 to 500°C, and a pressure ranging from 1 to 25 bar;
   ii) reverse-Water Gas Shift (rWGS), which comprises reacting CO2 and H2 to yield CO, followed by either a Low-Temperature Fischer-Tropsch-Synthesis, which comprises reacting CO and H2 in the presence of a cobalt-based catalyst, at a temperature ranging from 200 to 240°C, and a pressure ranging from 20 to 40 bar, to yield methane, C2-C100 linear alkanes; or High-Temperature Fischer-Tropsch-Synthesis, which comprises reacting CO2 and H2 in the presence of an iron-based catalyst at a temperature ranging from 300 to 400°C, and a pressure ranging from 10 to 25 bar to yield methane, C2-C20 alkanes, C2-C10 olefins, and oxygenates; and
   iii) methanol synthesis, which comprises reacting CO2 and H2 at a temperature ranging from 200 to 300 °C and at a pressure ranging from 35 to 100 bar, to yield methanol.
   wherein each of the reactions i), ii) and iii) produce the corresponding product, and a waste gas comprising hydrogen and carbon dioxide;
b) feeding the waste gas, a first amount of a carbon dioxide-containing feed gas, and oxygen-containing gas to an oxyfuel combustor;
c) combusting waste gas, the first amount of a carbon dioxide-containing feed gas, and oxygen-containing gas in the oxyfuel combustor to produce a combustion gas;
d) recovering heat from the combustion in the oxyfuel combustor with a heat recovery system;
e) feeding a second amount of a carbon dioxide-containing feed gas and combustion gas to a carbon capturing unit comprising an adsorption and a desorption column, the adsorption and desorption columns are in fluid communication with each other, wherein the adsorption column comprises a base portion and a top portion; the adsorption column extends a height from the base portion to the top portion; the base portion corresponds to a half of the height of the adsorption column; a feed gas inlet and a combustion gas inlet are located in the base portion; and the feed gas inlet is located above the combustion gas inlet in the base portion; the second amount of a carbon dioxide-containing feed gas is inputted through the feed gas inlet; and the combustion gas inputted through the combustion gas inlet;
f) adsorbing carbon dioxide in the adsorption column, and desorbing carbon dioxide in the desorption column, and producing carbon dioxide-containing synthesis gas; and
g) feeding the carbon dioxide synthesis from the desorption column, and a hydrogen-containing gas, to the one or more synthesis reactors.

### Brief Description of Drawings

FIG. 1 shows an embodiment of a system for the production of methanol according to the invention
FIG. 2 shows an embodiment of a system for the production of methanol according to the invention
FIG. 3 shows a configuration of methanol synthesis reactors of an embodiment of a system according to the invention.
FIG. 4 shows a configuration of the adsorption column of an embodiment of a system according to the invention wherein the base portion corresponds to a half of the height of the adsorption column.
FIG. 5 shows a configuration of the adsorption column of an embodiment of a system according to the invention wherein the base portion corresponds to a third of the height of the adsorption column.
FIG. 6 shows the molar flow, MF, in kmol/h, (FIG. 6A) and mol fraction, M, (FIG. 6B) of carbon dioxide in the overhead stream of examples Ex. 1 to Ex.6, as defined in the detailed description.
FIG. 7 shows the molar flow, MF, in kmol/h, (FIG. 7A) and mol fraction, M, (FIG. 7B) of methane in the overhead stream of examples Ex. 1 to Ex.6, as defined in the detailed description.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

For the purposes of the invention, any ranges given include both the lower and the upper endpoints of the range. Ranges given, such as temperatures, quantities, times, sizes, and the like, should be considered approximate, unless specifically stated.

As used herein, the term "waste gas" refers to a mixture of gases comprising unreacted gases, such as hydrogen and carbon dioxide, and/or by-products resulting from the production of methane, C2-C100 linear alkanes, C2-C10 olefins, oxygenates, methanol and combinations thereof. Equivalent terms that can be used instead of "waste gas" are "flue gas" or "exhaust gas".

The term "fluid entrance" refers to means for inputting a fluid, e.g., a gas, liquid or mixture thereof, within an apparatus, unit and/or system. A fluid entrance may comprise one or more inlets, and when the fluid entrance comprises more than one inlet, they may be distributed in different locations of the apparatus, unit and/or system and still belong to the same fluid entrance. Additionally, if a fluid entrance is physically distant from the apparatus, unit and/or system, the fluid entrance can be in fluid communication with the apparatus, unit and/or system by means of, for instance, a conduit, duct or pipe.

The term "in fluid communication" implies that there is a physical connection by which fluids can flow between two entities, such as apparatuses, units and/or systems. Suitable physical connections for connecting two entities are, for instance, conduits, ducts or pipes.

The term "carbon capturing unit" refers to a system configured to separate carbon dioxide from fluid mixtures. The separation is based on a two-step process involving adsorbing CO₂ onto a solid or liquid medium and then regenerating the medium by desorbing the CO ₂ in the desorption column. The carbon capturing unit can also be called "carbon dioxide capturing unit".

The term "carbon dioxide-containing feed gas" refers to feed gas which comprises carbon dioxide.

The term "feed gas" refers to a gas which is to be fed to an apparatus, unit and/or system according to the invention for being processed or converted into a gas having different composition and/or other distinct chemical products. The feed gas according to the invention may itself have been treated or conditioned previously to being fed to the apparatus, unit and/or system according to the invention.

The term "carbon dioxide-containing feed gas" refers to a feed gas containing carbon dioxide.

The term "raw gas" or "feedstock gas" refers to a feed gas, obtained by processes such as anaerobic digestion or gasification, and which has not been previously treated or conditioned within the system or process according to the invention. Suitable examples of raw gas or feedstock gas include biogas, syngas or mixtures thereof.

The term "oxygen-containing gas" refers to a gas wherein the oxygen is the main component of the gas. Preferably, the content of oxygen of the oxygen-containing gas is from 30 to 100% by volume.

The term "carbon dioxide-containing synthesis gas" refers to a gas containing carbon dioxide suitable for being used together with hydrogen for the synthesis of the products according to the invention.

The term "inert gas" refers to a gas that does not react within the system according to the invention and consequently may accumulate, e.g., in the adsorption column, if it is not purged or extracted from the system.

The term "non-reactant" refers to a substance that does not take part in and does not undergo chemical change during the synthesis reactions according to the invention.

The term "biogas" refers to a type of renewable gas obtained from organic waste from industries such as food, agriculture and livestock. As an example, biogas may comprise from 50 to 75% by volume of methane, from 25 to 50% by volume of carbon dioxide, and from 0.1 to 8% by volume of nitrogen.

The term "syngas" refers to a gaseous fuel obtained from carbon-rich substances (hard coal, coal, coke, naphtha, biomass) subjected to a high-temperature chemical process. As an example, syngas may comprise from 30 to 60% by volume of carbon monoxide, from 25 to 30% by volume of hydrogen, from 0 to 5% by volume of methane, and from 5 to 15% by volume of carbon dioxide.

The term "heat recovery system" refers to a system configured to capture and reuse thermal energy that would otherwise be wasted in a primary system, unit, or apparatus. Generally, a heat recovery system works by transferring heat from one system to another where it can be effectively used, thereby improving energy efficiency, reducing fuel consumption, and minimizing environmental impact.

The term "in thermal communication with" when referring to two apparatuses/systems is intended to mean that heat is transferred from one apparatus/system to another by suitable heat transfer means. The transfer of heat may be embodied by suitable heat transfer means, such as conducts or pipes, and suitable transfer medium, such as liquid or gas.

The term "oxyfuel combustor" refers to an apparatus configured for burning fuels, waste gases, flue gases or other combustible gases with a high-content oxygen gas (30-100% by volume) instead of air.

The term "combustion gas outlet" refers to an outlet configured to output combustion gas. The term "combustion gas inlet" refers to an inlet configured to input combustion gas. The term "feed gas inlet" refers to an inlet configured to input feed gas. The term "methanol outlet" refers to an outlet configured to output methanol. The term "carbon dioxide outlet" refers to an outlet configured to output carbon dioxide. The term "synthetized product outlet" refers to an outlet configured to output a product which has been synthetized in the one or more synthesis reactors. The synthetized fluid may be methane, C2-C100 linear alkanes, C2-C10 olefins, oxygenates, methanol and combinations thereof. The term "oxygenates" refers to products obtained with the system or process according to the invention which have undergone an oxidation reaction within the system. Such oxygenates may refer to C2-C100 linear alkanes or C2-C10 olefins, a carbon atom of which has been oxidized to form a hydroxyl, aldehyde, ketone or carboxylic acid functional group. Suitable examples of C2-C100 linear alkanes, C2-C10 olefins, and oxygenates are:
C2-C100 Linear Alkanes: Ethane, Propane, Butane, Pentane, Hexane, Heptane, Octane, Nonane, Decane, Dodecane, Tetradecane, Hexadecane, Octadecane, Eicosane, Docosane, Tetracosane, Hexacosane, Octacosane, and Triacontane.

C2-C10 Olefins: Ethene, Propene, 1-Butene, 2-Butene, 1-Pentene, 2-Pentene, 1-Hexene, 2-Hexene, 3-Hexene, 1-Heptene, 2-Heptene, 1-Octene, 2-Octene, 1-Nonene, 2-Nonene, 1-Decene, 2-Decene, 3-Decene, Cyclopentene, and Cyclohexene.

Oxygenates: Ethanol, 1-Propanol, 2-Propanol, 1-Butanol, 2-Butanol, Formaldehyde, Acetaldehyde, Acetone, Ethylene oxide, Propylene oxide, Acetic acid, Propanoic acid, Butyric acid, 2-Methylpropanol, 2,2-Dimethylpropanol, Octanol, 2-Octanone, Decanal, and 2,2,4-Trimethylpentanol.

The term "waste gas outlet" refers to an outlet configured to output waste gas from a unit, reactor, column or system. The term "oxygen-containing gas outlet" refers to an outlet configured to output oxygen-containing gas from an electrolyzer. The term "hydrogen outlet" refers to an outlet configured to output hydrogen gas from an electrolyzer. The term "overhead outlet" refers to an outlet configured to output methane and/or nitrogen from the carbon capturing unit, particularly from the adsorption column. All outlets and inlets described herein may be additionally configured to output or input, respectively, a different fluid from that to which are referred.

The "first fluid entrance" can also be called "oxyfuel combustor fluid entrance". The "second fluid entrance" can also be called "reactor fluid entrance". The "fluid exit" can also be called "gas conditioning unit fluid exit".

The term "mol fraction" refers to the amount, in moles, of a particular component in a mixture divided by the total amount, in moles, of the given mixture.

The term "molar flow" refers to the amount of a single component, in moles, that flows through a specific spatial location or element per unit of time. Unless otherwise indicated, the molar flow is expressed in kmol/hour (kmol/h).

As used herein, "% by volume" or "% v/v" of a component refers to the volume of the single component relative to the total volume of the composition or, if specifically mentioned, of another component.

In a first aspect, the invention provides a system for the production of a product selected from the group consisting of methane, C2-C100 linear alkanes, C2-C10 olefins, oxygenates, methanol and combinations thereof comprising:
- an oxyfuel combustor comprising a combustion gas outlet, and a first fluid entrance configured to input a carbon dioxide-containing feed gas, a waste gas and an oxygen-containing gas;
- a heat recovery system configured to recover heat from the oxyfuel combustor;
- a carbon capturing unit comprising
   - an adsorption column which comprises a combustion gas inlet in fluid communication with the combustion gas outlet of the oxyfuel combustor, a feed gas inlet configured to input carbon dioxide-containing feed gas, and an overhead outlet configured to output methane and/or nitrogen; and
   - a desorption column which comprises a carbon dioxide outlet configured to either output carbon dioxide-containing synthesis gas or a mixture of carbon dioxide-containing synthesis gas and hydrogen-containing gas;
wherein the adsorption and desorption columns are in fluid communication with each other, the adsorption column is configured to adsorb carbon dioxide, and the desorption column is configured to desorb carbon dioxide;
- one or more synthesis reactors, wherein the one or more synthesis reactors comprise:
   - a synthetized product outlet;
   - a waste gas outlet in communication with the first fluid entrance; and
   - a second fluid entrance configured to input a carbon dioxide-containing synthesis gas and a hydrogen-containing gas, and being in fluid communication with the carbon dioxide outlet;
wherein the adsorption column comprises a base portion and a top portion; the adsorption column extends a height from the base portion to the top portion; the base portion corresponds to one half of the height of the adsorption column; the feed gas inlet and the combustion gas inlet are located in the base portion; and the feed gas inlet is located above the combustion gas inlet in the base portion.

The feed gas inlet located above the combustion gas inlet in the base portion means that the combustion gas inlet is in a lower part of the base portion with respect to the feed gas inlet. This has been surprisingly shown to lead to a reduction of methane (CH₄) or nitrogen (N₂) dissolved in an adsorption medium while increasing the amount of CO₂ dissolved in the adsorption medium, i.e., increasing the selectivity towards CO₂. As a result, the amount of adsorption medium required for carbon capturing may be reduced, while the amount of CH₄ and other inert gases, such as nitrogen, that can contaminate the desorption is minimized. The CH₄ and/or N₂ which is not adsorbed within the adsorption medium can then be extracted or purged from the system through the overhead stream outlet (which can also be referred to as purge gases outlet).

As explained above, the system according to the invention allows the reduction of methane and nitrogen dissolved in the adsorption medium while increasing the amount of CO₂ dissolved in the adsorption medium. However, the methane and nitrogen may accumulate within the system and thus, the carbon capturing unit comprising an overhead outlet allows to conduct methane and nitrogen out of the system, avoiding these gases from recirculating within the system and reducing energy consumption that would be required for heating, compressing and/or transporting such gases through the system. Since the selectivity towards CO₂ is increased, the methane which is not adsorbed has also greater purity.

Advantageously, the system according to the invention comprises a first fluid entrance which is configured to input or conduct the carbon dioxide-containing feed gas. In state-of-the-art systems for the production of products such as methane, C2-C100 linear alkanes, C2-C10 olefins, oxygenates, methanol and combinations thereof, the synthesis of these products is exclusively carried out by using a carbon-dioxide containing feed gas, which may be previously submitted to carbon capturing. Contrary to these systems, the inventors have surprisingly found that if part of this carbon dioxide-containing feed gas is inputted to the oxyfuel combustor, its combustion allows to fulfil the energy requirements of the system while increasing the amount of CO₂ available, which in turn increases the partial pressure of CO₂ in the lower stages of the adsorption column and contributes to increasing CO₂ selectivity. In conventional systems, an additional energy source is generally needed for achieving the energetical requirements, and the source of CO₂ does not rely on the combustion of carbon-dioxide containing feed gas. Therefore, the system according to the invention advantageously obtains the energy required by other units or apparatus within the system by allowing to input part of the carbon dioxide-containing feed gas to the oxyfuel combustor, if needed, and profiting the thermal energy of the heat generated by combustion of said gases in combination with the waste gases coming from the synthesis reactor.

When it is indicated that the one or more synthesis reactors comprise a synthetized product outlet; a waste gas outlet in communication with the first fluid entrance; and a second fluid entrance in fluid communication with the carbon dioxide outlet, it is meant that:
- one of the reactors comprise the synthetized product outlet, the waste gas outlet and the second fluid entrance;
- each of the reactors comprises the synthetized product outlet, the waste gas outlet and the second fluid entrance; or
- alternatively, that the synthetized product outlet, the waste gas outlet and the second fluid entrance may be placed in different reactors.

This covers embodiments wherein, if more than one reactor is used, these reactors are in fluid communication with each other in such a way that one or more synthetized product outlets are combined; one or more waste gas outlets are in communication with the first fluid entrance; and one or more second fluid entrances are in fluid communication with the carbon dioxide outlet.

In a particular embodiment, the carbon capturing unit comprises a liquid adsorption medium. A liquid adsorption medium may be configured to adsorb carbon dioxide in the adsorption column and configured to desorb carbon dioxide in the desorption column when submitted to changes in a condition such as temperature, pressure, pH, and/or electrical energy.

In a particular embodiment, the desorption column comprises a hydrogen inlet configured to input a hydrogen-containing gas.

In known systems, the hydrogen outlet of the electrolyzer is in communication with the synthesis reactor where the synthesis takes place, since hydrogen is needed for the synthesis. The inventors have successfully implemented a desorption column comprising a hydrogen inlet, in such a way that the pressure generated by a stream of hydrogen-containing gas can be profited in the carbon capturing unit, particularly in the desorption column. This configuration is particularly advantageous since, although is not necessary to introduce the hydrogen-containing gas within the carbon capturing unit, it allows to attain the optimal pressure for the desorption of carbon dioxide within the carbon capturing unit. Then, a mixture of carbon dioxide-containing synthesis gas and hydrogen can be outputted through the carbon dioxide outlet and conducted to the second fluid entrance of the synthesis reactor.

In a particular embodiment, the system further comprises an electrolyzer comprising an oxygen-containing gas outlet in communication with the first fluid entrance, and a hydrogen outlet in communication to a hydrogen inlet of the desorption column, the second fluid entrance of the one or more synthesis reactors, or both.

In a particular embodiment, the base portion corresponds to one third of the height of the adsorption column. It has surprisingly been found by the inventors that if the base portion amounts to one third of the height of the adsorption column, i.e., the feed gas inlet and the combustion gas inlet are located in the lower third of the column, the molar fraction and molar flow of carbon dioxide in the overhead stream of the column, i.e., the stream to be outputted from the adsorption column, is significantly reduced. This is associated with an increase of selectivity of the system towards carbon dioxide capture.

In a particular embodiment, the adsorption column is configured to adsorb carbon dioxide by decreasing temperature in the liquid adsorption medium. In a particular embodiment, the desorption column is configured to desorb carbon dioxide by increasing temperature in the liquid adsorption medium. In a particular embodiment, the temperature of the liquid adsorption medium within the adsorption column ranges from -50°C to 0°C, and the temperature in of the liquid adsorption medium within the desorption column is 50°C to 200°C.

In a particular embodiment, the adsorption column of the carbon capturing unit is further configured to absorb carbon dioxide. In a particular embodiment, the adsorption column is further configured to absorb carbon dioxide. In this particular embodiment, the carbon capturing unit may further comprise an absorption medium for absorbing carbon dioxide. In a particular embodiment, the absorption medium comprises an amine solvent. In a particular embodiment, the amine solvent is selected from the group consisting of monoethanolamine (MEA), diglycolamine (DGA), diethanolamine (DEA), diisopropanolamine (DIPA), methyldiethanolamine (MDEA), 2-amino-2-methyl-1-propanol (AMP), MEA-MDEA blends, AMP-MDEA blends, amino acid-based solvents, and amine-functionalized ionic liquids.

In a particular embodiment, the adsorption comprises reversible physical adsorption. The physical adsorption, or physisorption, is driven by Van der Waal forces where the gas (e.g., CO2) and the adsorbent interacts in the adsorbent surface. In a particular embodiment, the adsorption consists of physical adsorption.

In a particular embodiment, the heat recovery system is selected from the group consisting of heat recovery steam generators (HRSGs), gas-to-gas heat exchangers, steam reheat systems, thermal oil heat exchangers, and combinations thereof.

HRSGs are commonly used to recover waste heat from high-temperature exhaust gases to generate steam. This steam can then be used in a steam turbine for power generation, or for other industrial heating processes.

Gas-to-gas heat exchangers transfer heat from hot exhaust gases to incoming fresh air or recycled exhaust gas. In oxyfuel systems, they are particularly useful for preheating oxygen or recycled CO₂, improving combustion efficiency and temperature control.

In a steam reheat system, exhaust gases from the oxyfuel combustor are used to reheat partially expanded steam from a high-pressure steam turbine before sending it to a low-pressure turbine.

Thermal oil heat exchangers transfer heat from high-temperature exhaust gases to a heat-transfer oil. The heated oil can then be used for various industrial processes, including space heating, drying, and preheating feedwater in different systems.

In a particular embodiment, the system further comprises a gas conditioning unit which comprises a raw gas inlet configured to input a raw gas selected from the group consisting of biogas, syngas or mixtures thereof; and a fluid exit in communication with the first fluid entrance and the feed gas inlet. Therefore, the raw gas may be conditioned in the gas conditioning unit, e.g., filtered and compressed, and be supplied to the oxyfuel combustor and to the adsorption column of the carbon capturing unit as a carbon dioxide-containing feed gas. As stated above, supplying the carbon dioxide-containing feed gas to the oxyfuel combustor and to the adsorption column allows to provide a carbon dioxide-containing synthesis gas to the one or more synthesis reactors while attaining the energy requirements of the system without the need of external energy sources.

In a particular embodiment, the conditioning unit further comprises a compressor and a filtering system. In a particular embodiment, the conditioning unit further comprises a drying system.

In a particular embodiment, the product is methanol, the top portion of the adsorption column comprises a methanol inlet; and the synthetized product outlet is in fluid communication with the methanol inlet. This embodiment advantageously allows methanol to act as adsorption medium within the carbon capturing unit. Therefore, in addition to producing methanol with the system according to the invention, the methanol itself can be used for adsorbing and desorbing carbon dioxide.

In a particular embodiment, the desorption column comprises a desorbed methanol outlet configured to output methanol, and the desorbed methanol outlet is in fluid communication either with a storage unit, a distillation system, or both.

In a particular embodiment, the storage unit comprises one or more storage containers configured to contain a product having equal or different purities. The one or more containers may be in fluid communication with a distillation system in such a way that the product is distilled and re-stored in a different container.

In a particular embodiment, where the product is methanol, the distillation system comprises one or more distillation columns in fluid communication with either the storage unit, the synthetized product outlet, and/or the desorbed methanol outlet, and wherein optionally, the one or more distillation columns comprise a distillation waste gas outlet in communication with the first fluid entrance. This allows to distill the methanol directly obtained from the synthesis reaction, the methanol stored in the storage unit and/or the methanol exiting the desorption column of the carbon capturing unit. In a particular embodiment, the synthetized product outlet is in fluid communication with the storage unit.

In a particular embodiment, the one or more distillation columns comprise a distillation waste gas outlet in communication with the first fluid entrance. A distillation waste gas outlet in communication with the first fluid entrance allows to conduct waste gas from distillation to the oxyfuel combustor for combusting them with the waste gas from the one or more synthesis reactors, the oxygen containing gas and the carbon dioxide containing feed gas, improving the energy efficiency of the system while reducing the amount of gases and greenhouse gases which are purged or flared to the atmosphere.

In a particular embodiment, the heat recovery system is in thermal communication with either the one or more synthesis reactors, the distillation system, or both. Thermal communication allows to transfer thermal energy from the heat recovery system to other apparatus or systems of the system for the production of the products according to the invention. In a particular embodiment, the heat recovery system may comprise heat transfer means for transferring heat to the synthesis reactor, the distillation system, or both. In a particular embodiment, the heat recovery system may comprise means for transforming thermal energy into electrical energy. In a particular embodiment, the heat recovery system may comprise means for storing electrical energy.

In a particular embodiment, the overhead outlet is in fluid communication with a methane storage system. This allows to store methane for other uses and thus, obtaining the products according to the invention and additional methane with the system.

In a particular embodiment, the combustion gas outlet is in fluid communication with the first fluid entrance. That is to say, part of the combustion gas may be recirculated to the oxyfuel combustor. The recirculation of combustion gas permits controlling the combustion temperature, heat capacity and convective heat transfer.

In a particular embodiment, the system further comprises one or more compressors. In a particular embodiment, the system further comprises one or more gas separators.

In a particular embodiment, the system is for the production of methanol, and comprises:
- an oxyfuel combustor comprising a combustion gas outlet, and a first fluid entrance configured to input a carbon dioxide-containing feed gas, a waste gas and an oxygen-containing gas;
- a heat recovery system configured to recover heat from the oxyfuel combustor;
   - a carbon capturing unit comprising
   - an adsorption column which comprises a combustion gas inlet in fluid communication with the combustion gas outlet of the oxyfuel combustor, a feed gas inlet configured to input carbon dioxide-containing feed gas, and an overhead outlet configured to output methane and/or nitrogen, and a methanol inlet; and
   - a desorption column which comprises a desorbed methanol outlet configured to output methanol, a carbon dioxide outlet configured to output either carbon dioxide-containing synthesis gas or a mixture of carbon dioxide-containing synthesis gas and hydrogen, and a hydrogen inlet;
wherein the adsorption and desorption columns are in fluid communication with each other, the adsorption column is configured to adsorb carbon dioxide, and the desorption column is configured to desorb carbon dioxide;
- one or more methanol synthesis reactors, wherein the one or more methanol synthesis reactors comprise:
   - a synthetized product outlet is in fluid communication with the methanol inlet;
   - a waste gas outlet in communication with the first fluid entrance; and a second fluid entrance in fluid communication with the carbon dioxide outlet; and
- an electrolyzer comprising an oxygen-containing gas outlet in communication with the first fluid entrance, and a hydrogen outlet in communication to either the hydrogen inlet, the second fluid entrance of the one or more methanol synthesis reactors, or both;
wherein the adsorption column comprises a base portion and a top portion; the adsorption column extends a height from the base portion to the top portion; the base portion corresponds to one third of the height of the adsorption column; the feed gas inlet (303) and the combustion gas inlet are located in the base portion; and the feed gas inlet is located above the combustion gas inlet in the base portion.

As mentioned above, the second aspect of the invention provides a for the production of a product selected from the group consisting of methane, C2-C100 linear alkanes, C2-C10 olefins, oxygenates, methanol and combinations thereof; from a carbon-dioxide synthesis gas and a hydrogen-containing gas, comprising:
a) conducting in one or more synthesis reactors, a reaction selected from the group consisting of:
   i) methanation, which comprises reacting H2 and CO2 to yield methane, at a temperature ranging from 250 to 500°C, and a pressure ranging from 1 to 25 bar;
   ii) reverse-Water Gas Shift (rWGS), which comprises reacting CO2 and H2 to yield CO, followed by either a Low-Temperature Fischer-Tropsch-Synthesis, which comprises reacting CO and H2 in the presence of a cobalt-based catalyst, at a temperature ranging from 200 to 240°C, and a pressure ranging from 20 to 40 bar, to yield C2-C100 linear alkanes; or High-Temperature Fischer-Tropsch-Synthesis, which comprises reacting CO2 and H2 in the presence of an iron-based catalyst at a temperature ranging from 300 to 400°C, and a pressure ranging from 10 to 25 bar to yield methane, C2-C20 alkanes, C2-C10 olefins, and oxygenates; and
   iii) Methanol synthesis, which comprises reacting CO2 and H2 at a temperature ranging from 200 to 300 °C and at a pressure ranging from 35 to 100 bar, to yield methanol.
   wherein each of the reactions i), ii) and iii) produce the corresponding product, and a waste gas comprising hydrogen and carbon dioxide;
b) feeding the waste gas, a first amount of a carbon dioxide-containing feed gas, and oxygen-containing gas to an oxyfuel combustor;
c) combusting waste gas, the first amount of a carbon dioxide-containing feed gas, and oxygen-containing gas in the oxyfuel combustor to produce a combustion gas;
d) recovering heat from the combustion in the oxyfuel combustor with a heat recovery system;
e) feeding a second amount of a carbon dioxide-containing feed gas and combustion gas to a carbon capturing unit comprising an adsorption and a desorption column, the adsorption and desorption columns are in fluid communication with each other, wherein the adsorption column comprises a base portion and a top portion; the adsorption column extends a height from the base portion to the top portion; the base portion corresponds to a half of the height of the adsorption column; a feed gas inlet and a combustion gas inlet are located in the base portion; and the feed gas inlet is located above the combustion gas inlet in the base portion; the second amount of a carbon dioxide-containing feed gas is inputted through the feed gas inlet; and the combustion gas inputted through the combustion gas inlet;
f) adsorbing carbon dioxide in the adsorption column, and desorbing carbon dioxide in the desorption column, and producing carbon dioxide-containing synthesis gas; and
g) feeding the carbon dioxide synthesis from the desorption column, and a hydrogen-containing gas, to the one or more synthesis reactors.

Typically, methanation involves the reaction of carbon dioxide (CO₂) with hydrogen (H₂) to produce methane (CH₄) and water (H₂O); the reverse-Water Gas Shift (rWGS) serves to convert carbon dioxide (CO₂) into carbon monoxide (CO) using hydrogen (H₂) and is primarily used to produce CO as a feedstock for further chemical processes like Fischer-Tropsch; Low-temperature Fischer-Tropsch synthesis involves converting a mixture of CO and H₂ into long-chain hydrocarbons; and High-temperature Fischer-Tropsch synthesis also converts involves converting a mixture of CO and H₂ into hydrocarbons but emphasizes lighter products.

In a particular embodiment, the process for the production a product selected from the group consisting of methane, C2-C100 linear alkanes, C2-C10 olefins, oxygenates, methanol and combinations thereof, from a carbon-dioxide synthesis gas and a hydrogen-containing gas, is carried out in a system according to any of the embodiments of the first aspect of the invention or combinations thereof.

In a particular embodiment, adsorbing carbon dioxide is carried out through physisorption.

In a particular embodiment, the product obtained in step a) is methanol, and the process further comprises feeding the product to the adsorption column for adsorbing carbon dioxide in the product, and desorbing carbon dioxide from the product in the desorption column.

In a particular embodiment, the base portion corresponds to a third half of the height of the adsorption column.

In a particular embodiment, the method further comprising conditioning raw gas in a conditioning unit to produce carbon dioxide-containing feed gas, wherein the raw gas is selected from the group consisting of biogas, syngas or mixtures thereof.

In a particular embodiment, wherein conditioning raw gas comprises filtering and compressing raw gas to produce carbon dioxide-containing feed gas.

In a particular embodiment, further comprising discharging product from the one or more synthesis reactors to either a distillation system, a storage unit, or both.

In a particular embodiment, the method further comprises distilling methanol discharged from the carbon capturing unit, the methanol storage unit, and/or the methanol synthesis reactor, in one or more distillation columns.

In a particular embodiment, the process further comprises conveying heat recovered from the oxyfuel combustor to the one or more methanol synthesis reactors or the distillation system.

In a particular embodiment, the method further comprises outputting methane from the carbon capturing unit and storing methane in a methane storage system.

In a particular embodiment, the carbon dioxide-containing synthesis gas and hydrogen-containing gas to a methanol synthesis reactor are fed to the methanol synthesis reactor at a pressure ranging from 5 to 30 bar.

In a particular embodiment, methanol synthesis comprises reacting dioxide-containing synthesis gas and the hydrogen-containing gas is carried out in the presence of a copper-based catalyst. Suitable copper-based catalysts are made of mixtures of CuO/ZnO/Al₂O₃, Cu/ZnO, CuO/ZrO₂, CuO/ZnO/ZrO₂ and Cu/SiO₂.

In a particular embodiment, methanation reaction comprises reacting H2 and CO2 to yield methane in the presence of Nickel-based catalysts (such as Ni/Al₂O₃ or Ni/SiO₂) or Cobalt-based catalysts (such as Co/Al₂O₃).

In a particular embodiment, rWGS comprises reacting CO2 and H2 to yield CO in the presence of a Cobalt-based catalysts (such as Co/Al₂O₃), a Copper-based catalysts (such as Cu/ZnO/Al₂O₃), Iron-based catalysts (such as Fe₂O₃), or Ruthenium-based catalysts (such as Ru/Al₂O₃ or Ru/CeO₂).

In a particular embodiment, Low-Temperature Fischer-Tropsch-Synthesis comprises reacting CO and H2 in the presence of a cobalt-based catalyst such as Co/SiO₂, Co/Al₂O₃.

In a particular embodiment, High-Temperature Fischer-Tropsch-Synthesis, which comprises reacting CO2 and H2 in the presence of an iron-based catalyst such as Fe/K₂O/Cu.

In a particular embodiment, the waste gas is fed to the oxyfuel combustor at a pressure ranging from 1 to 100 bar.

In a particular embodiment, the first and second amounts of carbon dioxide-containing feed gas are fed at pressure ranging from 10 to 26 bar.

In a particular embodiment, the oxyfuel combustor operates at a pressure between 1 to 15 bar.

In a particular embodiment, the process further comprises recirculating part of the combustion gas back to the oxyfuel combustor.

In a particular embodiment, the concentration of oxygen in the oxygen-containing gas is from 30 to 100% by volume, preferably 50 to 100% by volume, more preferably 70 to 100% by volume, even more preferably 80 to 100% by volume. In a particular embodiment, the oxygen-containing gas is pure oxygen.

In a particular embodiment, the concentration of carbon dioxide in the waste gas ranges from 50 to 90% by volume, and the concentration of hydrogen ranges from 5 to 95% by volume.

In a particular embodiment, the process further comprises obtaining distillation waste gas from the one or more distillation columns and feeding the distillation waste gas to the oxyfuel combustor. In a particular embodiment, distillation waste gas (i.e., waste gas obtained from the distillation of methanol) is obtained at a pressure ranging from 1 to 6 bar.

In a particular embodiment, adsorbing carbon dioxide in the adsorption column comprises decreasing temperature in a liquid adsorption medium, and desorbing carbon dioxide in the desorption column comprises increasing temperature in the liquid adsorption medium. In a particular embodiment, the temperature of the liquid adsorption medium within the adsorption column ranges from -50°C to 0°C, and the temperature in of the liquid adsorption medium within the desorption column is 10°C to 50°C.

In a particular embodiment, methanol is used in the adsorption and desorption columns for adsorbing and desorbing, respectively, carbon dioxide.

In a particular embodiment, adsorbing carbon dioxide in the adsorption column comprises increasing pressure in the liquid adsorption medium, and desorbing carbon dioxide in the desorption column comprises decreasing pressure in the liquid adsorption medium.

In a particular embodiment, the pressure at which the liquid medium is submitted in the within the adsorption column ranges from 20 to 40 bar, and the pressure at which the liquid medium is submitted in the within the desorption column ranges from 1 to 10 bar.

In a particular embodiment, the process further comprises absorbing carbon dioxide in the adsorption column. In this particular embodiment, the carbon capturing unit further comprises an absorption medium for adsorbing carbon dioxide. Therefore, in this embodiment, the process comprises adsorbing and absorbing carbon dioxide within the adsorption column.

In a particular embodiment, the process further comprises converting heat to electrical energy. Excess quantities of heat may be utilized using e.g. high-speed steam turbines driving generators or rotating equipment, depending on distance, power and specific plant size. In a particular embodiment, the process comprises converting heat to electrical energy in an electric steam generator.

The second aspect of the invention can also be reformulated as process for the production of a product selected from the group consisting of methane, C2-C100 linear alkanes, C2-C10 olefins, oxygenates, methanol and combinations thereof; from a carbon-dioxide synthesis gas and a hydrogen-containing gas, comprising:
a) conducting in one or more synthesis reactors, a reaction selected from the group consisting of:
   i) methanation, which comprises reacting H2 and CO2 to yield methane, at a temperature ranging from 250 to 500°C, and a pressure ranging from 1 to 25 bar;
   ii) reverse-Water Gas Shift (rWGS), which comprises reacting CO2 and H2 to yield CO, followed by either a Low-Temperature Fischer-Tropsch-Synthesis, which comprises reacting CO and H2 in the presence of a cobalt-based catalyst, at a temperature ranging from 200 to 240°C, and a pressure ranging from 20 to 40 bar, to yield methane, C2-C100 linear alkanes; or High-Temperature Fischer-Tropsch-Synthesis, which comprises reacting CO2 and H2 in the presence of an iron-based catalyst at a temperature ranging from 300 to 400°C, and a pressure ranging from 10 to 25 bar to yield methane, C2-C20 linear alkanes, C2-C10 olefins, and oxygenates; and
   iii) Methanol synthesis, which comprises reacting CO2 and H2 at a temperature ranging from 200 to 300 °C and at a pressure ranging from 35 to 100 bar, to yield methanol.
   wherein each of the reactions i), ii) and iii) produce the corresponding product, and a stream of waste gas comprising hydrogen and carbon dioxide;
b) feeding the stream of waste gas, a first stream of a carbon dioxide-containing feed gas, and a stream of oxygen-containing gas to an oxyfuel combustor;
c) combusting waste gas, carbon dioxide-containing feed gas, and oxygen-containing gas in the oxyfuel combustor to produce a combustion gas;
d) recovering heat from the combustion in the oxyfuel combustor with a heat recovery system;
e) feeding a second stream of a carbon dioxide-containing feed gas and a combustion gas stream to a carbon capturing unit comprising an adsorption and a desorption column, the adsorption and desorption columns are in fluid communication with each other, wherein the adsorption column comprises a base portion and a top portion; the adsorption column extends a height from the base portion to the top portion; the base portion corresponds to a half of the height of the adsorption column; a feed gas inlet and a combustion gas inlet are located in the base portion; and the feed gas inlet is located above the combustion gas inlet in the base portion; the second amount of a carbon dioxide-containing feed gas is inputted through the feed gas inlet; and the combustion gas inputted through the combustion gas inlet;
f) adsorbing carbon dioxide in the adsorption column, and desorbing carbon dioxide in the desorption column, and producing carbon dioxide-containing synthesis gas; and
g) feeding a carbon dioxide synthesis stream from the desorption column, and a hydrogen-containing gas stream, to the one or more synthesis reactors.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular embodiments described herein.

### Examples

In these figures the same reference signs have been used to designate matching elements.

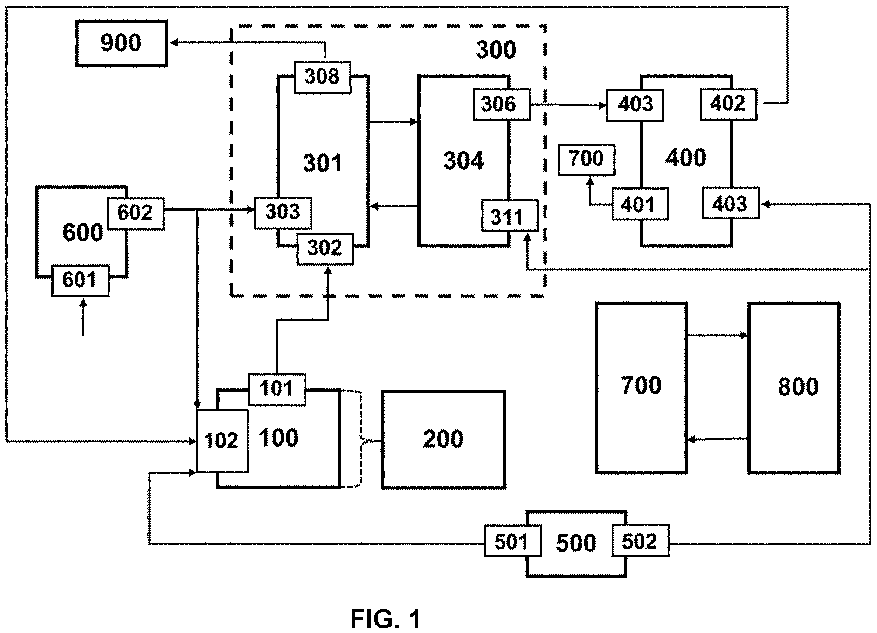

FIG.1 schematically represents an embodiment of a system according to the invention. As shown, the system for the production of a product selected from the group consisting of methane, C2-C100 linear alkanes, C2-C10 olefins, oxygenates, methanol and combinations thereof, comprises an oxyfuel combustor 100 comprising a combustion gas outlet 101, and a first fluid entrance 102 configured to input a carbon dioxide-containing feed gas, a waste gas and an oxygen-containing gas; a heat recovery system 200 configured to recover heat from the oxyfuel combustor; a carbon capturing unit 300 comprising an adsorption column 301 which comprises a combustion gas inlet 302 in fluid communication with the combustion gas outlet 101 of the oxyfuel combustor 100, a feed gas inlet 303 configured to input carbon dioxide-containing feed gas, and an overhead outlet 308 configured to output methane and/or nitrogen; and a desorption column 304 which comprises a carbon dioxide outlet 306 configured to either output carbon dioxide-containing synthesis gas or a mixture of carbon dioxide-containing synthesis gas and hydrogen-containing gas; wherein the adsorption and desorption columns are in fluid communication with each other, the adsorption column is configured to adsorb carbon dioxide, and the desorption column is configured to desorb carbon dioxide; one or more synthesis reactors 400, wherein the one or more synthesis reactors 400 comprise: a synthetized product outlet 401; a waste gas outlet 402 in communication with the first fluid entrance 102; and a second fluid entrance 403 configured to input a carbon dioxide-containing synthesis gas and a hydrogen-containing gas, and being in fluid communication with the carbon dioxide outlet 306; wherein the adsorption column 301 comprises a base portion 301a and a top portion 301b; the adsorption column 301 extends a height from the base portion 301a to the top portion 301b; the base portion 301a corresponds to one half of the height of the adsorption column 301; the feed gas inlet 303 and the combustion gas inlet 302 are located in the base portion 301a; and the feed gas inlet 303 is located above the combustion gas inlet 302 in the base portion 301a.

In the embodiment of FIG. 1, a stream of carbon dioxide-containing synthesis gas and hydrogen-containing gas are fed from the carbon dioxide outlet 306 of the desorption column 304 to the second fluid entrance 403 of the synthesis reactor through a duct or conduit. In other embodiments, a stream of carbon dioxide-containing synthesis gas and a stream of hydrogen-containing gas are fed from the carbon dioxide outlet 306 of the desorption column 304 to the second fluid entrance of the synthesis reactor through different ducts. In other embodiments, the carbon dioxide-containing synthesis gas is fed from the carbon dioxide outlet 306 of the desorption column 304 to the second fluid entrance 403 of the synthesis reactor 400 and the hydrogen-containing synthesis gas is fed to the second fluid entrance 403 of the synthesis reactor 400 directly from a hydrogen source, e.g., an electrolyzer.

Then, the carbon dioxide-containing synthesis gas and hydrogen-containing gas are reacted in the synthesis reactor 400. The reactions carried out by reacting the carbon dioxide from the carbon dioxide-containing synthesis gas and hydrogen from the hydrogen-containing gas are:
i) methanation, which comprises reacting H2 and CO2 to yield methane, at a temperature ranging from 250 to 500°C, and a pressure ranging from 1 to 25 bar;
ii) reverse-Water Gas Shift (rWGS), which comprises reacting CO2 and H2 to yield CO, followed by either a Low-Temperature Fischer-Tropsch-Synthesis, which comprises reacting CO and H2 in the presence of a cobalt-based catalyst, at a temperature ranging from 200 to 240°C, and a pressure ranging from 20 to 40 bar, to yield methane, C2-C100 linear alkanes; or High-Temperature Fischer-Tropsch-Synthesis, which comprises reacting CO2 and H2 in the presence of an iron-based catalyst at a temperature ranging from 300 to 400°C, and a pressure ranging from 10 to 25 bar to yield methane, C2-C20 alkanes, C2-C10 olefins, and oxygenates; and
iii) methanol synthesis, which comprises reacting CO2 and H2 at a temperature ranging from 200 to 300 °C and at a pressure ranging from 35 to 100 bar, to yield methanol.

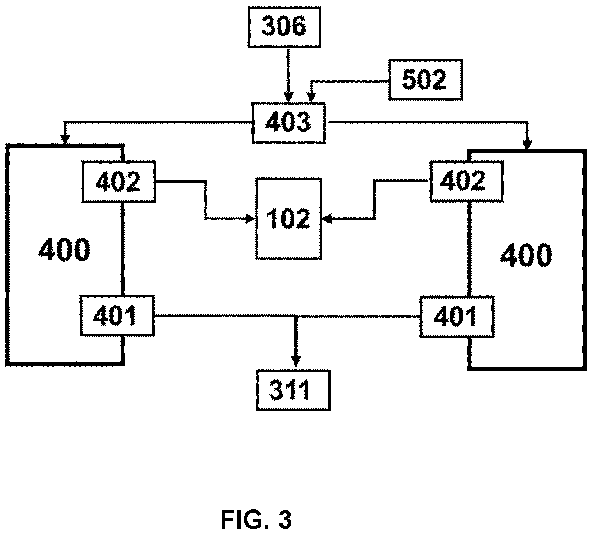

As shown in FIG. 3, the synthesis may also be carried out in more than one synthesis reactor. The reaction may be carried out simultaneously or sequentially in different synthesis reactors 400, which can be connected in parallel or in series. In case the reaction is carried out sequentially in different synthesis reactors 400 connected in series, one of the reactors may comprise the synthetized product outlet 401, the waste gas outlet 402 and the second fluid entrance 403; each of the reactors comprises a synthetized product outlet 401, a waste gas outlet 402 and a second fluid entrance 403; or alternatively that the synthetized product outlet 401, the waste gas outlet 402 and the second fluid entrance 403 may be placed in different reactors. The reactors can thus be in fluid communication with each other in such a way that the fluids which enter through the second fluid entrance(s) 403 are input to the one or more reactors; those which exit through the synthetized product outlet(s) 401 are conducted out of the reactor to a storage unit 700, to an inlet of the adsorption column 301 (e.g., methanol inlet 310 when the product is methanol) or to a treating or distillation unit 800; and those which exit through the waste gas outlet(s) 402 are conducted through conduits to the first fluid entrance 102.

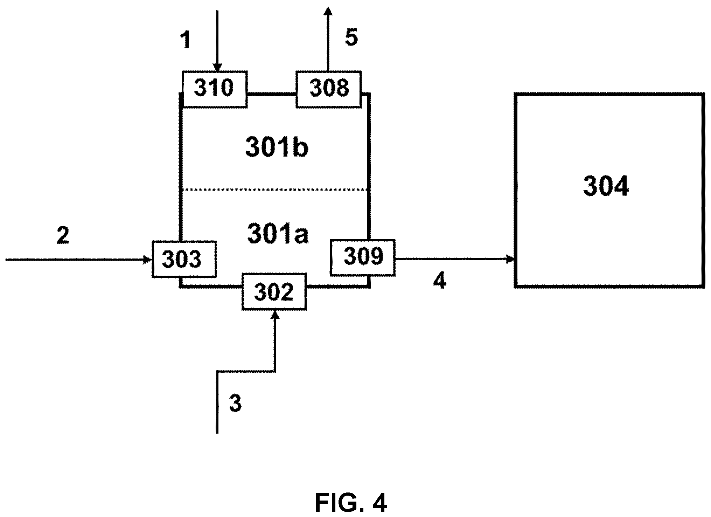

By way of example, FIG. 3 shows a configuration wherein two reactors comprise a second fluid entrance 403 in fluid communication with the carbon dioxide outlet 306 and the hydrogen outlet 502. It would also be possible that each of the reactors has its own second fluid entrance in communication with the carbon dioxide outlet 306 and the hydrogen outlet 502. Also in FIG. 4, each of the two reactors comprises a waste gas outlet 402 in communication with the first fluid entrance 102. Other configurations may be envisaged as long as one waste gas outlet is in communication with the first fluid entrance 102. For instance, it would be possible that a waste gas outlet 402 of one of the reactors is in fluid communication with the second reactor rather than with the first fluid entrance 102, and the waste gas outlet 402 of the second reactor is in communication with the first fluid entrance 102. The same can be applied *mutatis mutandis* for the synthetized product outlets 401 to conduct the product out of the reactors, or to conduct the product to the methanol inlet 310 when the synthetized product is methanol.

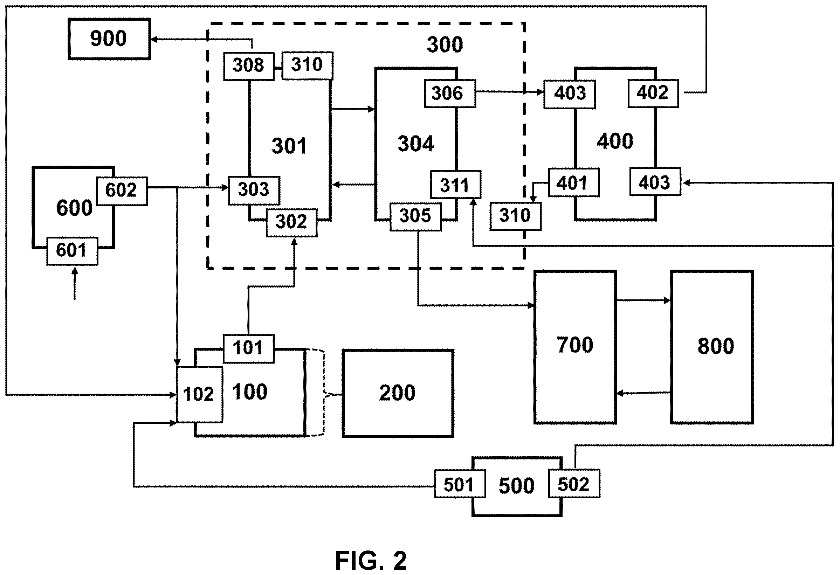

FIG. 2 shows an embodiment of a system according to the invention wherein the product is methanol, the top portion 301b of the adsorption column comprises a methanol inlet 310; and the synthetized product outlet 401 is in fluid communication with the methanol inlet 310. The methanol synthetized in the synthesis reactor 400 is conveyed to the methanol inlet 310 through a duct.

In embodiments of FIG. 1 and FIG. 2, the waste gas generated in the synthesis reactor 400 is fed to the first fluid entrance 102 of the oxyfuel combustor 100 as a waste gas stream through a conduit or duct. Additionally, a first amount of a carbon dioxide-containing feed gas and oxygen-containing gas are also fed to the oxyfuel combustor 100 through the first fluid entrance 102. The first fluid entrance 102 may comprise different inlets for conducting each of the fluids or streams or alternatively the fluids or streams may be mixed prior to be conducted to the first fluid entrance 102 or in the fluid entrance 102 itself. Then, the waste gas, the first amount of a carbon dioxide-containing feed gas, and the oxygen-containing gas are combusted in the oxyfuel combustor to produce a combustion gas. A combustion gas stream is conveyed from the combustion gas outlet 101 through a duct to the combustion gas inlet 302 of the adsorption column 301. In some embodiments, part of the combustion gas stream can be recirculated to the first fluid entrance 102 of the oxyfuel combustor 100 to control the temperature of a chamber of the oxyfuel combustor where the combustion takes place.

During the combustion in the oxyfuel combustor, the heat recovery system 200 recovers thermal energy from it. The thermal energy can be conveyed or transferred to other systems or apparatus of the system according to the invention, such as a distillation system 700, the one or more synthesis reactors 400, or both 700, 400. The thermal energy may in some embodiments be used for producing electrical energy, and afterwards storing the electrical energy and/or using it within the system.

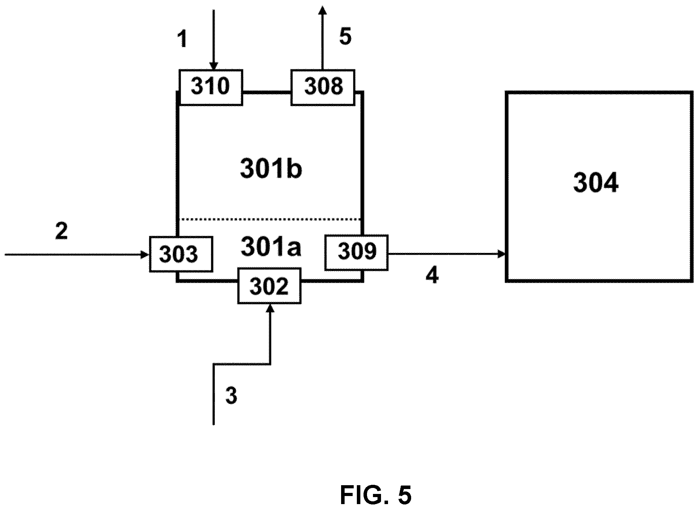

A stream containing a second amount of a carbon dioxide-containing feed gas and the combustion gas stream are fed to the adsorption column 301 through different ducts. As shown in the embodiment of FIG. 4, the adsorption column 301 comprises a base portion 301a and a top portion 301b; the adsorption column 301 extends a height from the base portion 301a to the top portion 301b; the base portion 301a corresponds to one half of the height of the adsorption column 301; the feed gas inlet 303 and the combustion gas inlet 302 are located in the base portion 301a; and the feed gas inlet 303 is located above the combustion gas inlet 302 in the base portion 301a. As discussed above, this specific configuration of the feed gas inlet 303 and the combustion gas inlet 302 improves the selectivity towards carbon dioxide within the system according to the invention. However, when the base portion 301a corresponds to one third of the height of the adsorption column 301 (e.g., FIG. 5) the selectivity towards carbon dioxide is further improved.

The production of a carbon dioxide-containing synthesis gas may be achieved by adsorbing carbon dioxide in the adsorption column, and desorbing carbon dioxide in the desorption column. The adsorption of carbon dioxide from the stream containing the second amount of a carbon dioxide-containing feed gas and the combustion gas stream is carried out by way of example, by using a liquid medium and reducing the temperature within the adsorption column. In the desorption column, the temperature is increased, and the carbon dioxide is desorbed from the liquid medium. In some embodiments, a liquid adsorption and/or absorption medium can be used for adsorbing and/or absorbing carbon dioxide. A solid adsorption medium for adsorbing carbon dioxide may also be used. In other embodiments, such as the one of FIG.2, the liquid medium is the methanol obtained in the synthesis reactor 400 and conducted through the synthetized methanol outlet to the methanol inlet 310, which in the top portion 301b of the adsorption column 301. After the methanol is desorbed in the desorption column 304, a methanol stream may be discharged from the desorption column through the desorbed methanol outlet 305. In the embodiment of FIG. 2, the desorption column comprises a desorbed methanol outlet 305 configured to output methanol, and the desorbed methanol outlet 305 is in fluid communication with a storage unit 700. However, the desorbed methanol outlet 305 may be in fluid communication with a distillation system 800, or simultaneously with the storage unit 700 and the distillation system. In some embodiments, the distillation system 800 may comprise one or more distillation columns in fluid communication with either the storage unit 700, the synthetized product outlet 401, and/or the desorbed methanol outlet 305. To contribute to the energy efficiency of the system, although not shown, the one or more distillation columns may comprise a distillation waste gas outlet in communication with the first fluid entrance 102.

Although optional, the embodiments of FIG. 1 and FIG. 2 additionally show a gas conditioning unit 600 which comprises a raw gas inlet 601 configured to input a raw gas selected from the group consisting of biogas, syngas or mixtures thereof; and a fluid exit 602 in communication with the first fluid entrance 102 and the feed gas inlet 303. Therefore, a stream of raw gas may be conveyed through a duct to a raw gas inlet 601 of the gas conditioning unit to condition or treat the stream of raw gas for producing the carbon dioxide-containing feed gas which will be conducted to the first fluid entrance 102 and the feed gas inlet 303. The raw gas may be for example biogas or syngas. Although not shown, the raw gas stream may be conditioned by filtering and compressing raw gas to produce the carbon dioxide-containing feed gas stream(s).

Several sources of hydrogen-containing gas and oxygen-containing gas may be used in the present invention. However, in the embodiments of FIG.1 and FIG.2 the source of these gases is water which has been electrolyzed in an electrolyzer 500. Therefore, in some embodiments, the system may further comprise an electrolyzer 500 comprising an oxygen-containing gas outlet 501 in communication with the first fluid entrance 102, and a hydrogen outlet 502 in communication with a hydrogen inlet 311 of the desorption column 304 and the second fluid entrance 403 of the one or more synthesis reactors 400. However, the system according to the invention also allows to connect the hydrogen outlet 502 only with the hydrogen inlet 311 of the desorption column 304 or the second fluid entrance 403 of the one or more synthesis reactors 400.

A methanol stream may be outputted from the desorption column through the desorbed methanol outlet 305, which is in fluid communication with a methanol storage unit 700, as shown in FIG. 2, but also can be in fluid communication directly with a distillation unit 800, or both 700, 800.

As shown in FIG. 2, in certain embodiments, the system may comprise a distillation system 800 comprising one or more distillation columns (not shown) in fluid communication with the methanol storage unit 700. However, the distillation system may also be in fluid communication with the synthetized product outlet 401, and/or the desorbed methanol outlet 305. The distillation system 800 in these embodiments allows to purify a methanol stream by distillation in the one or more distillation columns; the methanol stream discharged from the methanol synthesis reactor 400, from methanol which has been temporarily stored in the methanol storage unit 700 and/or the methanol stream coming from the desorption column 306 of the carbon capturing unit 300. The one or more distillation columns may also comprise a distillation waste gas outlet in communication with the first fluid entrance 102. This allows for a distillation waste gas stream for being conveyed to the oxyfuel combustor for being combusted and contribute to the energy efficiency of the system.

The carbon capturing unit 300, and particularly the adsorption column 301, further comprises an overhead outlet 308 configured to output methane and/or nitrogen from the carbon capturing unit 300, as shown in FIG. 1 and 2. Therefore, a methane and/or nitrogen stream can be outputted from the carbon capturing unit 300 through the overhead outlet 308 for reducing the amount of methane and/or nitrogen within the system, which lowers the energetic expenditure. Accordingly, the overhead outlet 308 which may be in fluid communication with a methane storage system 900 for storing the methane for other uses.

### Configuration of input and output streams in the adsorption column: Ex. 1 to 6

Different configurations of the input streams in the absorption column have been assessed by generating appropriate simulation models using the Aspen Plus software. The simulation models were calibrated with appropriate physical models to simulate the solubility of gases in methanol, water, and methanol-water mixtures accurately. Suitable column models were also calibrated through experimental data from the laboratory and pilot plant. Together, these improvements allowed for a reliable simulation that closely mirrors actual process performance, enhancing the overall fidelity and predictive capability of the process model.

An example (Ex. 3) of a configuration of the adsorption column tested in the simulations is shown in FIG. 4. In this example, the product obtained with the system is methanol, and this methanol is used as adsorption medium within the adsorption column and is inputted into the column through the methanol inlet 310. Briefly, the methanol inlet 310 is in the top portion 301b and in communication with the synthetized product outlet 401; the feed gas inlet 303 and the combustion gas inlet 302 are located in the base portion 301a; there is a carbon dioxide-containing methanol outlet 309 in fluid communication with the desorption column 304 by which methanol comprising carbon dioxide which has been adsorbed in the adsorption column 301 is conducted to the desorption column 304; and an overhead outlet 308 configured to output methane and inert gases, such as nitrogen, from the adsorption column. For the rest of examples tested in the simulations, the position of the feed gas inlet 303 and the combustion gas inlet 302 has been varied across the height of the column.

The different fluid streams being inputted or outputted from the adsorption column have been numbered from 1 to 5, as represented in FIG.4 (1: stream inputted at the methanol inlet 310; 2: stream inputted to the feed gas inlet 303, i.e., the carbon dioxide-containing feed gas; 3: stream inputted at the combustion gas inlet 302, i.e., the combustion gas; 4: stream outputted from the carbon dioxide-containing methanol outlet 309; and 5: stream outputted from the overhead outlet 308, i.e., overhead stream).

The mol fraction and molar flow of the input streams (1 to 3) have been defined as follows for all the examples:

| Stream | Parameter | CO₂ | CH₄ | H₂O | CH₃OH | N₂ |
|---|---|---|---|---|---|---|
| 1 | Molar flow (kmol/h) | - | - | 75.5 | 75.5 | - |
| | Mol fraction | - | - | 0.500 | 0.500 | - |
| 2 | Molar flow (kmol/h) | 0.664 | 1.233 | - | - | - |
| | Mol fraction | 0.350 | 0.650 | - | - | - |
| 3 | Molar flow (kmol/h) | 0.068 | - | - | - | 0.071 |
| | Mol fraction | 0.490 | - | - | - | 0.510 |

For defining the height of the adsorption column at which the input streams are inputted, the adsorption column has been conceptually divided in stages 0 to 100, each of the stages having the same height, in ascending order from the top to base of the column: the top portion 301b comprises stages 1 to 50 of the adsorption column; and the base portion 301a comprises stages 51 to 100 of the adsorption column.

As stated above, the position of the feed gas inlet 303 and the combustion gas inlet 302 has been varied across the adsorption column to observe the effect of different configurations of the inputted streams in the compositions of the overhead output stream. To this aim, six different examples (Ex. 1 to Ex. 6) having different configurations have been tested: Ex.1, the feed gas inlet 303 and the combustion gas inlet 302 at stage 100; Ex.2, the combustion gas inlet 302 at stage 67 and the feed gas inlet 303 at stage 100; Ex. 3, the combustion gas inlet 302 at stage 100 and the feed gas inlet 303 at stage 67; Ex. 4, the feed gas inlet 303 and the combustion gas inlet 302 at stage 67; Ex. 5, the combustion gas inlet 302 at stage 100 and the feed gas inlet 303 at stage 33; Ex. 6, the combustion gas inlet 302 at stage 100 and the feed gas inlet 303 at stage 17. Therefore, as shown in FIG. 4, the base portion 301a corresponds to one half of the height of the adsorption column 301. However, preferably, as shown in FIG. 5, the base portion 301a corresponds to one half of the height of the adsorption column 301.

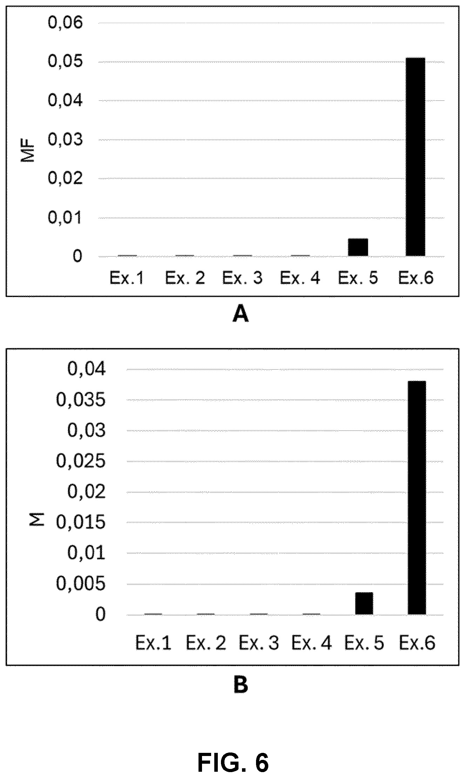

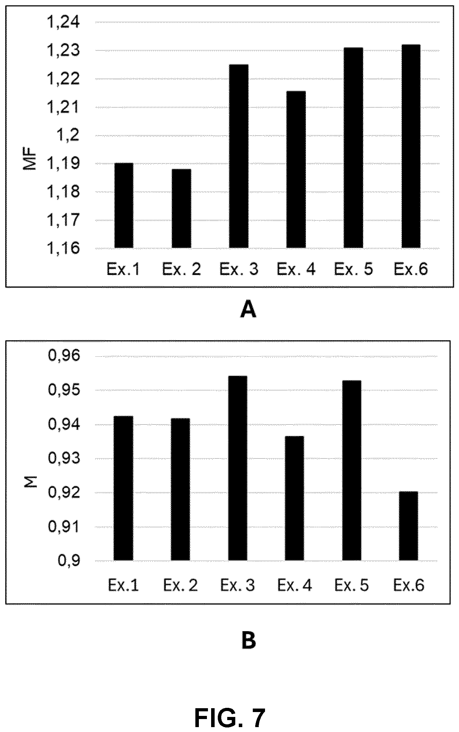

For all different examples, the mol fraction and molar flow (in kmol/hour) of carbon dioxide (FIG. 6) and methane (FIG. 7) has been calculated with the simulation program defined above; maintaining constant the mol fraction and molar flow of the input streams, as defined above. A low amount or flow of CO₂in the overhead stream 5 indicates high adsorption of CO₂ in the methanol within the adsorption column, which will be conducted through the carbon dioxide-containing methanol outlet 309 to the desorption column. A high amount of CH₄ in the overhead stream 5 indicates that the CH₄ is not adsorbed within the methanol and can be effectively separated from carbon dioxide and outputted from the adsorption column in the overhead stream through the overhead outlet 308.

FIG. 6A and FIG 6B show the molar flow and mol fraction of CO₂, respectively, in the overhead stream 5. In Ex. 1 to Ex. 3, the CO₂ mol fraction and molar flow in the overhead stream is zero, which indicates that all CO₂ is adsorbed in the methanol within the adsorption column and thus, having maximum CO₂ adsorption performance. In Ex. 4, the carbon dioxide-containing feed gas and combustion gas are introduced at the same stage (stage 67). In Ex. 4, the CO₂ composition is still minimized; however, this configuration lead to column instability, as it leaves the initial stages without sufficient gas flow at the start of operation. In Ex. 5 and Ex. 6, wherein carbon dioxide-containing feed gas is fed at stages 33 or 17, respectively, with combustion gas at stage 100, have shown the presence of CO₂ in the overhead and thus have a configuration leading to a worse CO₂ adsorption.

FIG. 7A and FIG. 7B show the molar flow and mol fraction of CH₄, respectively, in overhead stream 5. Surprisingly, the CH₄ molar fraction and flow is highest for Ex. 3, wherein carbon dioxide-containing feed gas is fed at stage 67 and the combustion gas at stage 100. The molar flow of CH₄ reaches its peak when carbon dioxide-containing feed gas is injected at stage 33 or 17 with combustion gas at stage 100. However, in these configurations, the CO₂ composition and molar flow in the overhead are higher than in other setups, which is not adequate for the performance of the system. Therefore, the most advantageous configuration is carbon dioxide-containing feed gas fed at stage 67 and combustion gas at stage 100. This setup achieves the highest CH₄ mol fraction and molar flow, while minimizing CO₂ molar fraction and mol flow, in the overhead stream 5.

Although these simulations have been carried out with methanol as liquid adsorption medium, and the best results are obtained for methanol as adsorption medium, adequate results are also obtained if instead of methanol, a different adsorption medium is used and is introduced through an inlet in the top portion of the adsorption column.

## Claims

1. A system for the production of a product selected from the group consisting of methane, C2-C100 linear alkanes, C2-C10 olefins, oxygenates, methanol and combinations thereof, comprising:
- an oxyfuel combustor (100) comprising a combustion gas outlet (101), and a first fluid entrance (102) configured to input a carbon dioxide-containing feed gas, a waste gas and an oxygen-containing gas;
- a heat recovery system (200) configured to recover heat from the oxyfuel combustor;
- a carbon capturing unit (300) comprising
∘ an adsorption column (301) which comprises a combustion gas inlet (302) in fluid communication with the combustion gas outlet (101) of the oxyfuel combustor (100), a feed gas inlet (303) configured to input carbon dioxide-containing feed gas, and an overhead outlet (308) configured to output methane and/or nitrogen; and
∘ a desorption column (304) which comprises a carbon dioxide outlet (306) configured to either output carbon dioxide-containing synthesis gas or a mixture of carbon dioxide-containing synthesis gas and hydrogen-containing gas;
wherein the adsorption and desorption columns are in fluid communication with each other, the adsorption column is configured to adsorb carbon dioxide, and the desorption column is configured to desorb carbon dioxide;
- one or more synthesis reactors (400), wherein the one or more synthesis reactors (400) comprise:
∘ a synthetized product outlet (401);
∘ a waste gas outlet (402) in communication with the first fluid entrance (102); and
∘ a second fluid entrance (403) configured to input a carbon dioxide-containing synthesis gas and a hydrogen-containing gas, and being in fluid communication with the carbon dioxide outlet (306);
wherein the adsorption column (301) comprises a base portion (301a) and a top portion (301b); the adsorption column (301) extends a height from the base portion (301a) to the top portion (301b); the base portion (301a) corresponds to one half of the height of the adsorption column (301); the feed gas inlet (303) and the combustion gas inlet (302) are located in the base portion (301a); and the feed gas inlet (303) is located above the combustion gas inlet (302) in the base portion (301a).

2. The system according to claim 1, wherein the product is methanol, the top portion (301b) of the adsorption column comprises a methanol inlet (310); and the synthetized product outlet (401) is in fluid communication with the methanol inlet (310).

3. The system according to claim 2, further comprising an electrolyzer (500) comprising an oxygen-containing gas outlet (501) in communication with the first fluid entrance (102), and a hydrogen outlet (502) in communication to a hydrogen inlet (311) of the desorption column (304), the second fluid entrance (403) of the one or more synthesis reactors (400), or both (311,403).

4. The system according to any of the claims 1-3, wherein the base portion (301a) corresponds to one third of the height of the adsorption column (301).

5. The system according to any of the claims 1-4, further comprising a gas conditioning unit (600) which comprises a raw gas inlet (601) configured to input a raw gas selected from the group consisting of biogas, syngas or mixtures thereof; and a fluid exit (602) in communication with the first fluid entrance (102) and the feed gas inlet (303).

6. The system according to any of the claims 2-5, wherein the desorption column comprises a desorbed methanol outlet (305) configured to output methanol, and the desorbed methanol outlet (305) is in fluid communication either with a storage unit (700), a distillation system (800), or both.

7. The system according to claim 6, wherein the distillation system (800) comprises one or more distillation columns in fluid communication with either the storage unit (700), the synthetized product outlet (401), and/or the desorbed methanol outlet (305), and wherein optionally, the one or more distillation columns comprise a distillation waste gas outlet in communication with the first fluid entrance (102).

8. A process for the production of a product selected from the group consisting of methane, C2-C100 linear alkanes, C2-C10 olefins, oxygenates, methanol and combinations thereof; from a carbon-dioxide synthesis gas and a hydrogen-containing gas, comprising:
a) conducting in one or more synthesis reactors, a reaction selected from the group consisting of:
i) methanation, which comprises reacting H2 and CO2 to yield methane, at a temperature ranging from 250 to 500°C, and a pressure ranging from 1 to 25 bar;
ii) reverse-Water Gas Shift (rWGS), which comprises reacting CO2 and H2 to yield CO, followed by either a Low-Temperature Fischer-Tropsch-Synthesis, which comprises reacting CO and H2 in the presence of a cobalt-based catalyst, at a temperature ranging from 200 to 240°C, and a pressure ranging from 20 to 40 bar, to yield methane, C2-C100 linear alkanes; or High-Temperature Fischer-Tropsch-Synthesis, which comprises reacting CO2 and H2 in the presence of an iron-based catalyst at a temperature ranging from 300 to 400°C, and a pressure ranging from 10 to 25 bar to yield methane, C2-C20 alkanes, C2-C10 olefins, and oxygenates; and
iii) Methanol synthesis, which comprises reacting CO2 and H2 at a temperature ranging from 200 to 300 °C and at a pressure ranging from 35 to 100 bar, to yield methanol.
wherein each of the reactions i), ii) and iii) produce the corresponding product, and a waste gas comprising hydrogen and carbon dioxide;
b) feeding the waste gas, a first amount of a carbon dioxide-containing feed gas, and oxygen-containing gas to an oxyfuel combustor;
c) combusting waste gas, the first amount of a carbon dioxide-containing feed gas, and oxygen-containing gas in the oxyfuel combustor to produce a combustion gas;
d) recovering heat from the combustion in the oxyfuel combustor with a heat recovery system;
e) feeding a second amount of a carbon dioxide-containing feed gas and combustion gas to a carbon capturing unit comprising an adsorption and a desorption column, the adsorption and desorption columns are in fluid communication with each other, wherein the adsorption column comprises a base portion and a top portion; the adsorption column extends a height from the base portion to the top portion; the base portion corresponds to a half of the height of the adsorption column; a feed gas inlet and a combustion gas inlet are located in the base portion; and the feed gas inlet is located above the combustion gas inlet in the base portion; the second amount of a carbon dioxide-containing feed gas is inputted through the feed gas inlet; and the combustion gas inputted through the combustion gas inlet;
f) adsorbing carbon dioxide in the adsorption column, and desorbing carbon dioxide in the desorption column, and producing carbon dioxide-containing synthesis gas; and
g) feeding the carbon dioxide synthesis from the desorption column, and a hydrogen-containing gas, to the one or more synthesis reactors.

9. The process according to claim 8, wherein adsorbing carbon dioxide is carried out through physisorption.

10. The process according to any of the claims 8-9, wherein the product obtained in step a) is methanol, and the process further comprises feeding the product to the adsorption column for adsorbing carbon dioxide in the product, and desorbing carbon dioxide from the product in the desorption column.

11. The process according to any of the claims 8-10, wherein the base portion corresponds to a third half of the height of the adsorption column.

12. The process according to any of the claims 8-11, further comprising conditioning raw gas in a conditioning unit to produce carbon dioxide-containing feed gas, wherein the raw gas is selected from the group consisting of biogas, syngas or mixtures thereof.

13. The process according to any of the claims 8-12, further comprising discharging product from the one or more synthesis reactors to either a distillation system, a storage unit, or both.

14. The process according to claim 13, further comprising conveying heat recovered from the oxyfuel combustor to the one or more synthesis reactors or the distillation system.

15. The process according to any of the claims 8-14, further comprising outputting methane from the carbon capturing unit, and storing methane in a methane storage system.
